**Europäisches Patentamt**

**European Patent Office**

**Office européen des brevets**

(19)

(11) Publication number : **0 380 157 B1**

(12) **EUROPEAN PATENT SPECIFICATION**

(45) Date of publication of patent specification :
29.04.92 Bulletin 92/18

(21) Application number : 90200104.9

(22) Date of filing : 15.01.90

(51) Int. Cl.$^5$ : **A61K 33/04,** A61K 33/30, A61K 35/04, // (A61K33/04, 31:045, 31:20, 31:62, 31:215, 31:17, 31:19), (A61K33/30, 33:04, 31:045, 31:20, 31:62, 31:215, 31:17, 31:19), (A61K35/04, 33:30, 33:04, 31:045, 31:20)

(54) **Wound healing powder.**

(30) Priority : 24.01.89 NL 8900165

(43) Date of publication of application :
01.08.90 Bulletin 90/31

(45) Publication of the grant of the patent :
29.04.92 Bulletin 92/18

(84) Designated Contracting States :
AT BE CH DE DK FR GB IT LI LU NL SE

(56) References cited :
US-A- 4 668 692

(56) References cited :
"Merck Index", 1983, edition 10, pages 39,1289,1408,1457,226,1200,345,1410,185, Merck & Co., Inc., Rahways, NY, US
PATENT ABSTRACTS OF JAPAN, vol. 12, no. 467 (C-550)[3314], 7th December 1988; & JP-A-63 188 628 (SHISEIDO CO. LTD) 04-08-1988

(73) Proprietor : **Weisberg, Yosef David**
**Rehov Shimon Hamaccabi 4**
**Jerusalem (IL)**

(72) Inventor : **Weisberg, Yosef David**
**Rehov Shimon Hamaccabi 4**
**Jerusalem (IL)**

(74) Representative : **Ellowicz, Leo et al**
**Octrooibureau Polak & Charlouis Laan Copes van Cattenburch 80**
**NL-2585 GD Den Haag (NL)**

## Description

The invention relates to a wound healing powder.

In comparison with other forms of pharmaceutical compositions, the number of various wound healing powders is relatively small. Most compositions for wound treatment are creams of unguents. The commercially available wound healing powders generally contain 1 or 2 active substances, sometimes on particular carriers. All these powders possess a drawback that they have little general applicability, or may be exclusively used by medically trained personnel, or that though they are safe, they only show a slight or dubious activity. In general the action of the powders is based on absorption of exuded moisture, wherein as a result of the presence of moisture possible therapeutical agents penetrate into the tissues through the liquid phase. The accessibility for therapeutic agents can be improved further by using keratolytic agents.

This invention provides a wound healing powder which is generally applicable and which is also suitable for household use in the case of small injuries and for wound healing in the case of minor operations.

The invention provides a wound healing powder which is characterized by the fact that it contains as an active ingredient 40-60% by weight of a combination of allantoin with at least one of the following compounds: sulphur, urea, sodium pyruvate, benzoyl peroxide an salicylic acid or a salt thereof as a keratolytic agent; at least one of the following compounds: zinc oxide, calamine and alum as an astringent agent; at least one of the following compounds: zinc-undecylenate, undecylenic acid and salicylic acid or a salt thereof as an antifungus agent; one or more of the following compounds; salicylic acid or a salt thereof and benzoyl peroxide as a bacteriostatic agent; and at least one hydroxy-benzoic ester as a preservative, wherein the total number of active compounds is at least 5.

All the active components of the present wound healing powder are known per se, but some are seldom or never used in a wound healing powder.

Allantoin is a known wound treating agent which possesses keratolytic and adstringent effects.

Sulphur has keratolytic activity and is also an antimycotic agent.

Salicylic acid or a salt thereof is a multifunctional agent, having keratolytic, fungicidal and bacteriostatic activities; this agent is particularly suitable as a component of the present wound healing powder.

Also benzoyl peroxide is multifunctional and possesses keratolytic and bacteriostatic activities.

Calamine, which may be used as an astringent agent, also contains iron and consequently causes some colouring of the powder. From an esthetic standpoint this may or may not be preferred. Alum, which is an astringent agent, has a rather strong activity and is not suitable under all circumstances.

In the past coal tar or wood tar has been used as an inflammation inhibiting agent in wound treating compositions. In principle such a tar can also be incorporated in the present powder, but in view of the known carcinogenic properties of tar this is not preferred, even when the skin is only contacted with a low concentration thereof during a short time.

For special purposes an antipruritic agent, for instance camphor or phenol, can be incorporated in the wound healing powder. Also boric acid, which is known to exert a soothing action, is a possible component of the present composition.

The mutual ratio's of the active components can be varied within broad limits, wherein however the zinc oxide (or calamine) is the principal ingredient which comprises 50-70% of the active component. Furthermore, it is desirable that the undecylenate comprises 25-35% by weight of the active mixture.

As mentioned already, all the above-discussed active ingredients are known per se. They are discussed for instance in the Merck Index, 10th edition, 1983.

As the carrier any carrier suitable for skin powders may be used, but because talc has a tendency to form lumps, when it is contacted with moisture, one uses preferably kaolin or a similar material. Furthermore, one may add colloidal $SiO_2$ in order to inhibit caking, as is also known per se.

According to a specially preferred embodiment the wound healing powder, based on the total composition, contains the following active substances: 3-5% allantoin, 1-2% sulphur, 10-18% zinc undecylenate, 0.7-1% salicylic acid, 24-32% zinc oxide and 0.1-0.2% ethyl and/or propyl hydroxybenzoate.

The following example serves to elucidate the invention.

A wound healing powder was prepared which contained the following ingredients.

2

|  | % by weight |
|---|---|
| Allantoin | 2 |
| Sulphur | 1.5 |
| Zinc undecylenate | 14 |
| Salicylic acid | 0.85 |
| Zincoxide | 28 |
| Sodium-ethyl- and | |
| sodium-propyl-hydroxybenzoate (1:1) | 0.14 |
| Kaolin | qs 100 |

Thereafter 2.5%, based on the above total, of colloidal $SiO_2$ was further incorporated in this mixture. This wound healing powder was tested in Jerusalem for the circumcision of a large number of babies. Normally some disinfecting composition is applied to the concerning small wound, in order to avoid inflammations (compare Genesis, XXXIV, 25). However, in practice not all babies can tolerate the usual compositions. With the present powder such problems did not occur, and the wounds healed at least as readily as in the case of other compositions.

Also in Israel a woman, due to an accident at home, incurred a rather serious burn on her leg. By chance a sample of the above mentioned powder was present in that house, the composition of which was not known to the inhabitants of this house. The wound was provisionally dusted with this powder, whereafter the woman went by taxi to the hospital for further treatment. When she arrived there and was examined, it appeared that the wound already had such a good appearance that the doctor who examined her did not consider further measures necessary.

In the fall of 1989 a diabetes patient in Israel had an open wound. This wound was treated with the above powder without the patient knowing its nature, and to the stupefaction of the patient himself the wound had closed within two days.

## Claims

1. Wound healing powder, **characterized** in that it contains as an active ingredient 40-60% by weight of a combination of allantoin with at least one of the following compounds: sulphur, urea, sodium pyruvate, benzoyl peroxide and salicylic acid or a salt thereof as a keratolytic agent; at least one of the following compounds: zinc oxide, calamine and alum as an astringent agent; at least one of the following compounds: zinc-undecylenate, undecylenic acid and salicylic acid or a salt thereof as an anti-fungus agent; one or more of the following compounds: salicylic acid or a salt thereof and benzoyl peroxide as a bacteriostatic agent; and at least one hydroxy benzoic ester as a preservative, wherein the total number of the active component is at least 5.

2. Wound healing powder according to claim 1, **characterized** by the fact that it contains sulphur.

3. Wound healing powder according to claim 1 or 2, **characterized** by the fact that it contains salicylic acid or a salt thereof as keratolytic, anti-fungus and bacteriostatic agent.

4. Wound healing powder according to claims 1-3, **characterized** by the fact that it contains coal tar as inflammation-inhibiting agent.

5. Wound healing powder according to claims 1-4, **characterized** by the fact that it contains an antipruritic agent and/or boric acid.

## Patentansprüche

1. pulver zur Heilung von Wunden, dadurch gekennzeichnet, dass es als aktiven Bestandteil 40-60 Gew.-% einer Kombination von Allantoin mit, als keratolytischem Mittel, zumindest einer der folgenden Verbindungen: Schwefel, Harnstoff, Natriumpyruvat, Benzoylperoxid und Salicylsäure oder ein Salz davon; als Adstringens, zumindest einer der folgenden Verbindungen: Zinkoxid, Galmei und Alaun; als fungizidem Mittel, zumindest einer der folgenden Verbindungen: Zinkundecylenat, Undecylensäure und Salicylsäure oder ein Salz davon; als bakteriostatischem Mittel, einer oder mehreren der folgenden Verbindungen: Salicylsäure oder ein Salz

3

davon und Benzoylperoxid; und als Konservierungsmittel zumindest einen Hydroxybenzoesäureester enthält, wobei die gesamte Anzahl der aktiven Komponenten zumindest gleich 5 ist.

2. Pulver zur Heilung von Wunden nach Anspruch 1, dadurch gekennzeichnet, dass es Schwefel enthält.

3. Pulver zur Heilung von Wunden nach Anspruch 1 oder 2, dadurch gekennzeichnet, dass es Salicylsäure oder ein Salz davon als keratolytisches, fungizides und bakteriostatisches Mittel enthält.

4. Pulver zur Heilung von Wunden nach den Ansprüchen 1-3, dadurch gekennzeichnet, dass es Steinkohlenteer als entzündungshemmendes Mittel enthält.

5. Pulver zur Heilung von Wunden nach den Ansprüchen 1-4, dadurch gekennzeichnet, dass es ein Mittel gegen Juckreiz und/oder Borsäure enthält.

## Revendications

1. Poudre cicatrisante caractérisée en ce qu'elle contient à titre de principe actif 40 à 60 % en poids d'une combinaison d'allantoïne avec au moins l'un des composés suivants : soufre, urée, pyruvate de sodium, peroxyde de benzoyle et acide salicylique ou un sel de celui-ci à titre d'agent kératolytique; au moins l'un des composés suivants : oxyde de zinc, calamine et alun à titre d'agent astringent; au moins l'un des composés suivants : undécylénate de zinc, acide undécylénique et acide salicylique ou un sel de celui-ci à titre d'agent antifongique; un ou plusieurs des composés suivants : acide salicylique ou un sel de celui-ci et du peroxyde de benzoyle à titre d'agent bactériostatique; et au moins un ester d'acide hydroxybenzoique à titre d'agent conservateur, le nombre total des principes actifs étant d'au moins cinq.

2. Poudre cicatrisante selon la revendication 1, caractérisée en ce qu'elle contient du soufre.

3. Poudre cicatrisante selon le revendication 1 ou 2, caractérisée en ce qu'elle contient de l'acide salicylique ou un sel de celui-ci à titre d'agent kératolytique, antifongique et bactériostatique.

4. Poudre cicatrisante selon les revendications 1-3, caractérisée en ce qu'elle contient du goudron de houille à titre d'agent anti-inflammatoire.

5. Poudre cicatrisante selon les revendications 1-4, caractérisée en ce qu'elle contient un agent antipruritique et/ou de l'acide borique.